# EUROPEAN PATENT APPLICATION

(11) **EP 4 082 567 A1**
(43) Date of publication of application: **02.11.2022**
(21) Application number: 21873704.7
(22) Date of filing: 25.08.2021
(51) Int. Cl.: A61K 39/215

(54) **INFLUENZA VIRUS VECTOR-BASED NOVEL CORONAVIRUS VACCINE AND PREPARATION METHOD THEREFOR**

(30) Priority: 15.03.2021 CN 202110275628
(71) Applicant: Guangzhou N Biomed Ltd., Guangzhou, Guangdong 510663 (CN)
(72) Inventor: CHEN, Ling, Guangdong 510663 (CN); HAN, Lujie, Guangdong 510663 (CN); YANG, Chenchen, Guangdong 510663 (CN); WANG, Qian, Guangdong 510663 (CN); LIU, Bo, Guangzhou, Guangdong 510663 (CN)
(74) Representative: Gassner, Birgitta
(86) International application number: PCT/CN2021/114572
(87) International publication number: WO 2022/193553

(57) **Abstract**

Disclosed are a novel coronavirus vaccine based on an influenza virus vector and a preparation method thereof. The vaccine can efficiently express two antigens, i.e., its own HA antigen and an exogenous SC2R1 antigen, enabling the vaccine to induce immune responses to the two antigens, thus achieving the purpose of preventing both influenza virus and novel coronavirus, thereby eliminating the impacts of the two major infectious diseases of influenza and novel coronavirus on social economy, etc. at one time. In addition, based on existing mature influenza platform techniques, the influenza vaccine can be prepared and produced on a large scale, and the use of influenza vaccines has a long history and good safety.

## Description

### TECHNICAL FIELD

The present disclosure relates to a novel coronavirus vaccine based on an influenza virus vector and a preparation method thereof.

### BACKGROUND

The novel coronavirus SARS-CoV-2 has a latent period up to 24 days and is highly contagious. Unlike viruses that cause SARS, some cases have infectivity during the latent period, while some other virus carriers do not show any obvious symptoms, thus increasing the difficulty of epidemic prevention and control. Currently, there have been no clearly verified specific antiviral drugs against novel coronavirus in China and other countries. Therefore, preventing and blocking the spread of the virus is the key of controlling the epidemic. In addition, the application of vaccines has played an irreplaceable role in eliminating a variety of infectious diseases.

Influenza, an acute respiratory infectious disease caused by influenza viruses, spreads rapidly, is highly contagious, and has a certain seasonality. Influenza viruses occasionally cause pandemics, causing severe respiratory diseases with a high mortality. The recombination of complete gene fragments of influenza virus results in the formation of a new subtype, and since the human population lacks immunity to mutant strains of the new subtype, a pandemic is caused. The occurrence of viral gene mutations and the emergence of new subtypes of the virus have both aroused people's concern about health issues. Influenza is not effectively controlled due to the emergence of drug resistance, and therefore, vaccination is the main strategy for the prevention and control of influenza.

### SUMMARY

A first object of the present disclosure is to provide a novel coronavirus vaccine based on an influenza virus vector.

A second object of the present disclosure is to provide a method for preparing a novel coronavirus vaccine based on an influenza virus vector.

The technical solutions used by the present disclosure are as follows:
A first aspect of the present disclosure provides a novel coronavirus vaccine based on an influenza virus vector, the vaccine comprising an influenza virus vector loaded with the gene SC2R1 that encodes an S protein receptor binding region of a novel coronavirus.

Preferably, the vaccine is a DNA plasmid or an RNA expression plasmid.

Preferably, the influenza virus vector comprises a truncated PB2, PB1, PA, NP, HA, NA, M or NS gene sequence, and the truncated gene sequence retains coding region packaging signal sequences at the 3' and 5' ends; and
the gene SC2R1 that encodes the S protein receptor binding region of the novel coronavirus is inserted into the truncated PB2, PB1, PA, NP, HA, NA, M or NS gene sequence.

Preferably, the gene SC2R1 that encodes the S protein receptor binding region of the novel coronavirus is inserted into an open reading frame of the truncated gene sequence of PB2, PB1, PA, NP, HA, NA, M or NS.

Preferably, the 3' end of the gene SC2R1 that encodes the S protein receptor binding region of the novel coronavirus is connected to a truncated fragment of NA gene, and the truncated fragment of the NA gene contains a coding region packaging signal at the 3' end.

NA is a second type of glycoprotein on the surface of an influenza virion and is a typical type II glycoprotein, with the amino terminus facing the interior of the virion. NA protein has a tetramer formed in its structure, and each monomer of the protein is composed of a short and non-conserved amino-terminal cytoplasmic region, a hydrophobic transmembrane region, a stem region, and a globular head. In the present disclosure, the 1st to 201st nucleotides at the 3' end is selected, and this segment contains a coding region packaging signal (183 nucleotides) at the 3' end of NA. This segment is connected to the gene SC2R1 that encodes the S protein receptor binding region of the novel coronavirus via a linker sequence, and the segment obtained after connection is then inserted into the truncated PB2, PB1, PA, NP, HA, NA, M or NS gene sequence. At this point, the 1st to 201st nucleotides of NA are used as a base to achieve the purpose of bringing the S protein receptor binding region of the novel coronavirus to the surface of the influenza virus.

The amino acid sequence encoded by the truncated fragment of the NA gene is: 
MNPNQKITTIGSICLVVGLISLILQIGNIISIWISHSIQTGSQNHTGICNQNIITYKNST
WVKDTTS (SEQ ID NO. 1);
 preferably, the nucleotide sequence of the truncated fragment of the NA gene is:

atgaatccaaatcagaaaataacaaccattggatcaatctgtctggtagtcggactaattagcctaatattgcaaatagggaatataat
ctcaatatggattagccattcaattcaaactggaagtcaaaaccatactggaatatgcaaccaaaacatcattacctataaaaatagcacctg
ggtaaaggacacaacttca (SEQ ID NO. 2).

Preferably, the gene SC2R1 that encodes the S protein receptor binding region of the novel coronavirus is connected to the truncated fragment of the NA gene via a linker sequence.

Commonly used linkers include GGGSG, GGGGS or GSG. Of course, other commonly used fusion protein linkers can also be used.

Preferably, the amino acid sequence of the S protein receptor binding region of the novel coronavirus is as set forth below:

RVQPTESIVRFPNITNLCPFGEVFNATRFASVYAWNRKRISNCVADYSVLYNSASFST
FKCYGVSPTKLNDLCFTNVYADSFVIRGDEVRQIAPGQTGKIADYNYKLPDDFTGCVIA
WNSNNLDSKVGGNYNYLYRLFRKSNLKPFERDISTEIYQAGSTPCNGVEGFNCYFPLQS
YGFQPTNGVGYQPYRVVVLSFELLHAPATVCGPKKSTNLVKNKCVNFNFNGLTGTGVL
TESNKKFLPFQQFGRDIADTTDAVRDPQTLEILDITPCS (SEQ ID NO. 3).

Preferably, the nucleotide sequence of the gene SC2R1 that encodes the S protein receptor binding region of the novel coronavirus is as set forth below:

agggtgcagccaacagagtccatcgtgcgctttcccaatatcaccaacctgtgcccttttggcgaggtgttcaatgccacacgcttc
gccagcgtgtacgcctggaataggaagcgcatctccaactgcgtggccgactattctgtgctgtacaacagcgcctccttctctacctttaa
gtgttatggcgtgagccccaccaagctgaatgatctgtgctttacaaacgtgtacgccgattccttcgtgatcaggggcgacgaggtgcgc
cagatcgcaccaggacagaccggcaagatcgcagactacaattataagctgcctgacgatttcacaggctgcgtgatcgcctggaactct
aacaatctggatagcaaagtgggcggcaactacaattatctgtaccggctgtttagaaagtctaatctgaagccattcgagcgggacatctc
caccgagatctaccaggccggctctacaccctgcaatggcgtggagggctttaactgttatttccctctgcagtcctacggcttccagccaa
ccaacggcgtgggctatcagccctacagagtggtggtgctgtcttttgagctgctgcacgcacctgcaaccgtgtgcggcccaaagaag
agcacaaatctggtgaagaacaagtgcgtgaacttcaacttcaacggactgaccggcacaggcgtgctgaccgagagcaacaagaagt
tcctgccatttcagcagttcggcagggacatcgcagataccacagacgccgtgcgcgaccctcagaccctggagatcctggacatcaca
ccatgttcc (SEQ ID NO. 4).

A protein expressed by the amino acid sequence of the S protein receptor binding region of the novel coronavirus is capable of, in the human body,
inducing an immune response; or
generating a biological reporter molecule; or
generating a trace molecule for detection; or
regulating gene function; or
functioning as a therapeutic molecule.

Preferably, the influenza virus vector is derived from an influenza A or B strain, preferably an H1N1 subtype influenza strain.

Preferably, the vaccine further comprises a pharmaceutically acceptable adjuvant, carrier, diluent or excipient.

A second aspect of the present disclosure provides a method for preparing a novel coronavirus vaccine based on an influenza virus vector, comprising the following steps:
(1) truncating at least one of PB2, PB1, PA, NP, HA, NA, M or NS genes of an influenza virus genome, retaining coding region packaging signal sequences at the 3' and 5' ends; and
(2) connecting the truncated fragment of the NA gene to the gene SC2R1 that encodes an S protein receptor binding region of a novel coronavirus, then inserting the fragment obtained after connection into the truncated PB2, PB1, PA, NP, HA, NA, M or NS gene sequence of an influenza virus to obtain the novel coronavirus vaccine based on the influenza virus vector;
wherein preferably, the truncated fragment of the NA gene comprises a coding region packaging signal at the 3' end.

Preferably, the amino acid sequence encoded by the truncated fragment of the NA gene is as set forth in SEQ ID NO. 1; and
preferably, the nucleotide sequence of the truncated fragment of the NA gene is as set forth in SEQ ID NO. 2.

Preferably, the amino acid sequence of the S protein receptor binding region of the novel coronavirus is as set forth in SEQ ID NO. 3.

Beneficial Effects of the disclosure: The present disclosure is a novel coronavirus vaccine based on an influenza virus vector, which can efficiently express two antigens, i.e., its own HA antigen and an exogenous SC2R1 antigen, enabling the vaccine to induce immune responses to the two antigens, thus achieving the purpose of preventing both influenza virus and novel coronavirus, thereby eliminating the impacts of the two major infectious diseases of influenza and novel coronavirus on social economy, etc. at one time. In addition, based on existing mature influenza platform techniques, the influenza vaccine can be prepared and produced on a large scale, and the use of influenza vaccines has a long history and good safety.

### BRIEF DESCRIPTION OF DRAWINGS

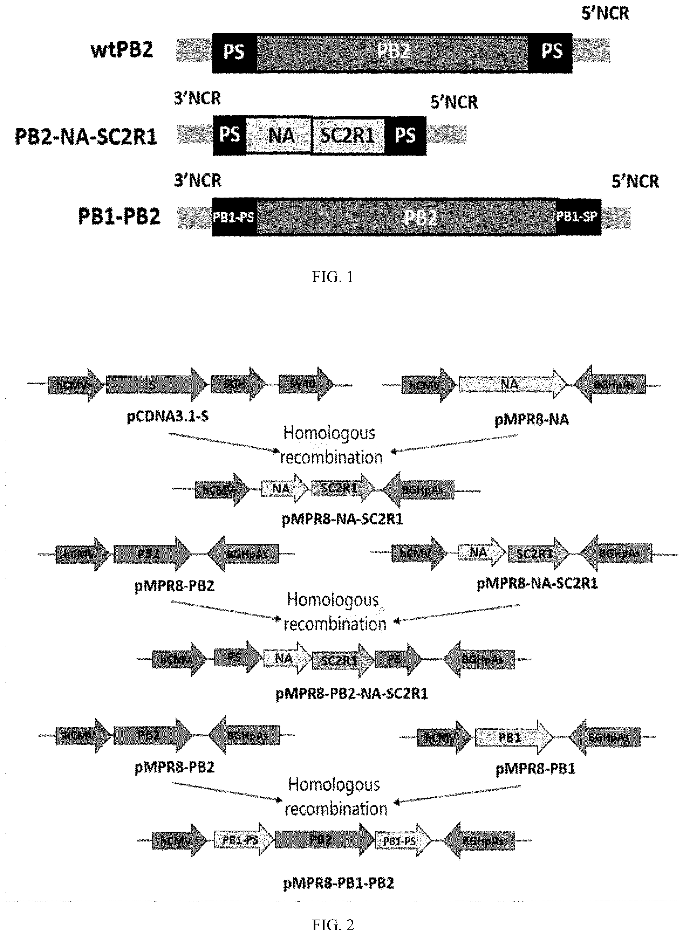

FIG. 1 is a schematic diagram of a vector construction design.
FIG. 2 is a flow chart of the construction of the vectors pMPR8-NA-SC2R1, pMPR8-PB2-NA-SC2R1 and pMPR8-PB1-PB2.

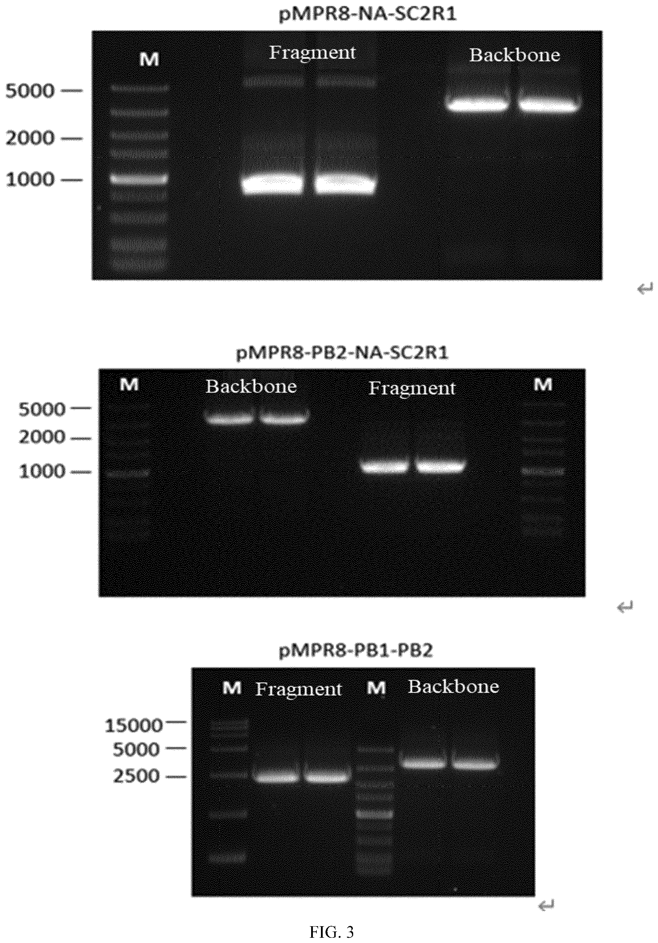

FIG. 3 is the PCR amplification results of the vectors pMPR8-NA-SC2R1, pMPR8-PB2-NA-SC2R1 and pMPR8-PB1-PB2 in terms of backbones and fragments.

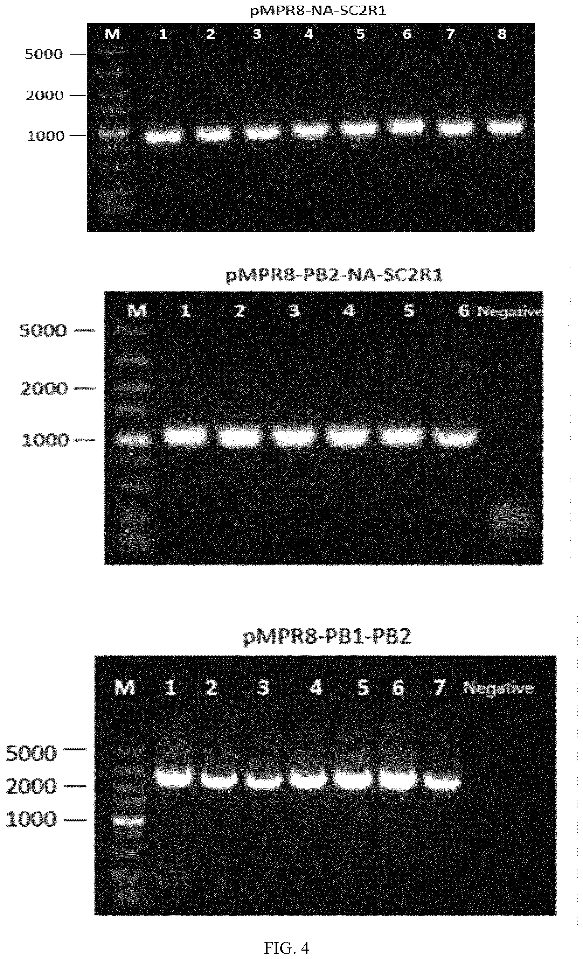

FIG. 4 is the PCR amplification identification results of the plasmids pMPR8-NA-SC2R1, pMPR8-PB2-NA-SC2R1 and pMPR8-PB1-PB2.
FIG. 5 is a schematic diagram of the rescue of influenza virus by means of a reverse genetics technique.
FIG. 6 is the verification results of SC2R1 protein expression of the virus PR8-NA-SC2R1.
FIG. 7 is the verification results of HA protein expression of the virus PR8-NA-SC2R1.
FIG. 8 is the ELISA results of SC2R1 antibody.
FIG. 9 is the HAI results of HA antibody.

### DETAILED DESCRIPTION

In order to understand the technical content of the present disclosure more clearly, the following examples are specifically given for detailed description in conjunction with the accompanying drawings. It is to be understood that these examples only serve to illustrate the present disclosure rather than limiting the scope of the present disclosure. The experimental methods in the following examples, in which no specific conditions are specified, are generally carried out in accordance with conventional conditions or the conditions recommended by the manufacturers. Various common chemical reagents used in the examples are all commercially available products.

The term "2019-nCoV" in the present disclosure is a novel coronavirus, which is highly infectious. Its proteins are divided into spike glycoprotein (S protein), envelope glycoprotein (E protein), membrane glycoprotein (M protein), and nucleocapsid protein (N protein). S protein (spike protein) is the most important surface membrane protein of coronavirus and contains two subunits, i.e. S1 and S2. S protein, which can recognize a host cell receptor and mediate membrane fusion, is essential for virion to enter into a cell, and is a key factor in viral infection of a host cell. S1 mainly contains a receptor binding region responsible for recognizing cell receptors. The receptor binding region directly participates in the recognition of a host receptor. Amino acid variations in this region lead to changes in the species-tropism and infection characteristics of the virus. The 331st to 524th amino acids of the S protein are known as receptor binding domain. In the present disclosure, the complete SC2R1 sequence of the receptor binding region, i.e. 319th to 591st amino acids of S, is selected, and by means of codon optimization, a base fragment of 819 bp is obtained, as set forth in SEQ ID NO. 4.

The term "influenza virus" in the present disclosure is a single negative strand RNA virus, expressing eight proteins including PB2, PB1, PA, NP, HA, NA, M, and NS. By means of binding to a cap structure of a host mRNA precursor molecule, the PB2 protein plays an important role in generating primers required for viral vRNA transcription and initiating the transcription process. PB2 protein is also involved in vRNA replication and is an important determinant of influenza virus pathogenicity. The full length of PB2 is 759 amino acids. After truncated as appropriate, PB2 possesses a better capacity of accommodating exogenous genes. NA is a second type of glycoprotein on the surface of an influenza virion and is a typical type II glycoprotein, with the amino terminus facing the interior of the virion. NA protein has a tetramer formed in its structure, and each monomer of the protein is composed of a short and non-conserved amino-terminal cytoplasmic region, a hydrophobic transmembrane region, a stem region, and a globular head. We have selected the 1st to 201st nucleotides at the N terminus, and this segment contains a coding region packaging signal (183 nucleotides) at the 3' end of NA. This segment is connected to SC2R1 via a linker sequence, and the segment obtained after connection is then inserted into the truncated PB2 gene sequence. At this point, the 1st to 201st nucleotides of NA are used as a base to achieve the purpose of bringing SC2R1 to the surface of the influenza virus.

### Example 1 Novel coronavirus vaccine based on influenza virus vector

As an example of the present disclosure, the influenza virus vector comprised a truncated PB2 gene sequence, wherein the truncated PB2 gene sequence retained packaging signals of 36 nucleotides at both the 3' and 5' ends respectively, and the other parts were all cut off; and the truncated fragment (SEQ ID NO. 2) of the NA gene was connected to the gene SC2R1 (SEQ ID NO. 4) that encoded the S protein receptor binding region of the novel coronavirus via a linker sequence, and then inserted into an open reading frame of the truncated PB2 gene sequence. The specific preparation method was as follows:

### 1 Construction of pMPR8-NA-SC2R1 plasmid

With pCDNA3.1-S plasmid (pCDNA3.1 was a high expression vector known in the art, S was the optimized S gene of SARS-CoV-2, which had been filed for a patent with the patent number being 202010110070.8, and the vector pCDNA3.1-S could be synthesized by a biological company) as a template and SC2R1-F and SC2R1-R as primers, PCR amplification was carried out to obtain an SC2R1 fragment (SEQ ID NO.4), and the fragment was subjected to gel extraction and purification; and with pMPR8-NA plasmid (pM was a high expression vector known in the art, NA could be found in NCBI with the gene sequence number being AF3 89120.1, and the vector pMPR8-NA could be synthesized by a biological company) as a template and NA-F and NA-R as primers, PCR amplification was carried out to obtain a PR8-NA backbone, and the backbone was subjected to gel extraction and purification. The NA fragment in the PR8-NA backbone was a truncated fragment of the NA gene, which contained a coding region packaging signal at the 3' end, and the sequence was as set forth in SEQ ID NO. 2. The SC2R1 fragment and the PR8-NA backbone fragment were subjected to homologous recombination with Exnase enzyme, and Top10 was transformed and inoculated into Amp-resistant LB solid medium to obtain cloned plasmid pMPR8-NA-SC2R1.
SC2R1-F: gtaaaggacacaacttcaggcagcggcagggtgcagccaacagagtccatctgtgc (SEQ ID NO. 5),
SC2R1-R: acccagaagcacggcctctttaggaacatggtgtgatgtccaggatctt (SEQ ID NO. 6),
PCR conditions: 98°C, 3 min; 98°C, 10 s; 60°C, 10 s; 72°C, 40 s; cycles 30; 72°C, 5 min; and 4°C, 10 min.
NA-F: taaagaggccgtgcttctgggttgaattaatcaggggacgaccta (SEQ ID NO. 7),
NA-R: tgaagttgtgtcctttacccaggtgctatttttataggta (SEQ ID NO. 8),
PCR conditions: 98°C, 3 min; 98°C, 10 s; 60°C, 20 s; 72°C, 40 s; cycles 30; 72°C, 5 min; and 4°C, 10 min.

### 2 PCR identification of the plasmid pMPR8-NA-SC2R1

SC2R1-F: gtaaaggacacaacttcaggcagcggcagggtgcagccaacagagtccatctgtgc (SEQ ID NO. 9),
SC2R1-R: acccagaagcacggcctctttaggaacatggtgtgatgtccaggatctt (SEQ ID NO. 10),
PCR conditions: 98°C, 3 min; 98°C, 10 s; 60°C, 10 s; 72°C, 40 s; cycles 30; 72°C, 5 min; and 4°C, 10 min.

### 3 Construction of pMPR8-PB2-NA-SC2R1 plasmid

With pMPR8-NA-SC2R1 plasmid as a template and PB2-NA(TY)-F and PB2-NA-SC2R1-R as primers, PCR amplification was carried out to obtain an NA-SC2R1 fragment, and the fragment was subjected to gel extraction and purification; and with pMPR8-PB2 plasmid (pM was a high expression vector known in the art, PB2 could be found in NCBI with the gene sequence number being AF389115.1, and the vector pMPR8-PB2 could be synthesized by a biological company) as a template and PB2-F and PB2-R as primers, PCR amplification was carried out to obtain a PR8-PB2 backbone, and the backbone was subjected to gel extraction and purification. The PB2 gene in the obtained PR8-PB2 backbone was truncated, wherein only packaging signals of 36 nucleotides were retained at both the 3' and 5' ends. The NA-SC2R1 fragment and the PR8-PB2 backbone fragment were subjected to homologous recombination with Exnase enzyme, and Top10 was transformed and inoculated into Amp-resistant LB solid medium to obtain cloned plasmid pMPR8-PB2-NA-SC2R1.
PB2-NA(TY)-F: ctaagaaatctaatatcgatgaatccaaatcagaaaataacaaccattggatca (SEQ ID NO. 11),
PB2-NA-SC2R1-R: aattcttttggtcgctgttttaggaacatggtgtgatgtccaggatctcc (SEQ ID NO. 12),
PCR conditions: 98°C, 3 min; 98°C, 10 s; 50°C, 5 s; 72°C, 35 s; cycles 30; 72°C, 5 min; and 4°C, 10 min.
PB2-F: acagcgaccaaaagaattcggatggccatcaattagtgtcga (SEQ ID NO. 13),
PB2-R: cgatattagatttcttagttcttttattctttctatattgaatata (SEQ ID NO. 14),
PCR conditions: 98°C, 3 min; 98°C, 10 s; 50°C, 15 s; 72°C, 35 s; cycles 30; 72°C, 5 min; and 4°C, 10 min.

### 4 PCR identification of the plasmid pMPR8-PB2-NA-SC2R1

PB2-NA(TY)-F: ctaagaaatctaatatcgatgaatccaaatcagaaaataacaaccattggatca (SEQ ID NO. 15),
PB2-NA-SC2R1-R: aattcttttggtcgctgttttaggaacatggtgtgatgtccaggatctcc (SEQ ID NO. 16),
PCR conditions: 98°C, 3 min; 98°C, 10 s; 50°C, 5 s; 72°C, 35 s; cycles 30; 72°C, 5 min; and 4°C, 10 min.

### 5 Construction of pMPR8-PB1-PB2 plasmid

With pMPR8-PB2 plasmid as a template and PB2-P5-F and PB2-P6-R as primers, PCR amplification was carried out to obtain a PB2 fragment, and the fragment was subjected to gel extraction and purification. With pMPR8-PB 1 plasmid (pM was a high expression vector known in the art, PB1 could be found in NCBI with the gene sequence number being CY148249.1, and the vector pMPR8-PB1 could be synthesized by a biological company) as a template and PB2-P7-F and PB2-P7-R as primers, PCR amplification was carried out to obtain a PR8-PB1 backbone, and the backbone was subjected to gel extraction and purification. The PB2 fragment and the PR8-PB1 backbone fragment were subjected to homologous recombination with Exnase enzyme, and Top10 was transformed and inoculated into Amp-resistant LB solid medium to obtain cloned plasmid pMPR8-PB1-PB2.
PB2-P5-F: cacaaaatgctataagcacaatggaaagaataaaag (SEQ ID NO. 17),
PB2-P6-R: ttcatgatctcagtgaactcctaattgatggccatccga (SEQ ID NO. 18),
PCR conditions: 98°C, 3 min; 98°C, 10 s; 56°C, 15 s; 72°C, 30 s; cycles 30; 72°C, 5 min; and preservation at 12°C.
PB2-P7-F: cttttattctttccattgtgcttatagcattttgtg (SEQ ID NO. 19),
PB2-P8-R: tcggatggccatcaattaggagttcactgagatcatgaa (SEQ ID NO. 20),
PCR conditions: 98°C, 3 min; 98°C, 10 s; 56°C, 15 s; 72°C, 30 s; cycles 30; 72°C, 5 min; and preservation at 12°C.

### 6 PCR identification of the plasmid pMPR8-PB1-PB2

PB2-P5-F: cacaaaatgctataagcacaatggaaagaataaaag (SEQ ID NO. 21),
PB2-P6-R: ttcatgatctcagtgaactcctaattgatggccatccga (SEQ ID NO. 22),
PCR conditions: 98°C, 3 min; 98°C, 10 s; 56°C, 15 s; 72°C, 30 s; cycles 30; 72°C, 5 min; and preservation at 12°C.

### 7 Rescue of influenza virus by means of reverse genetics technique

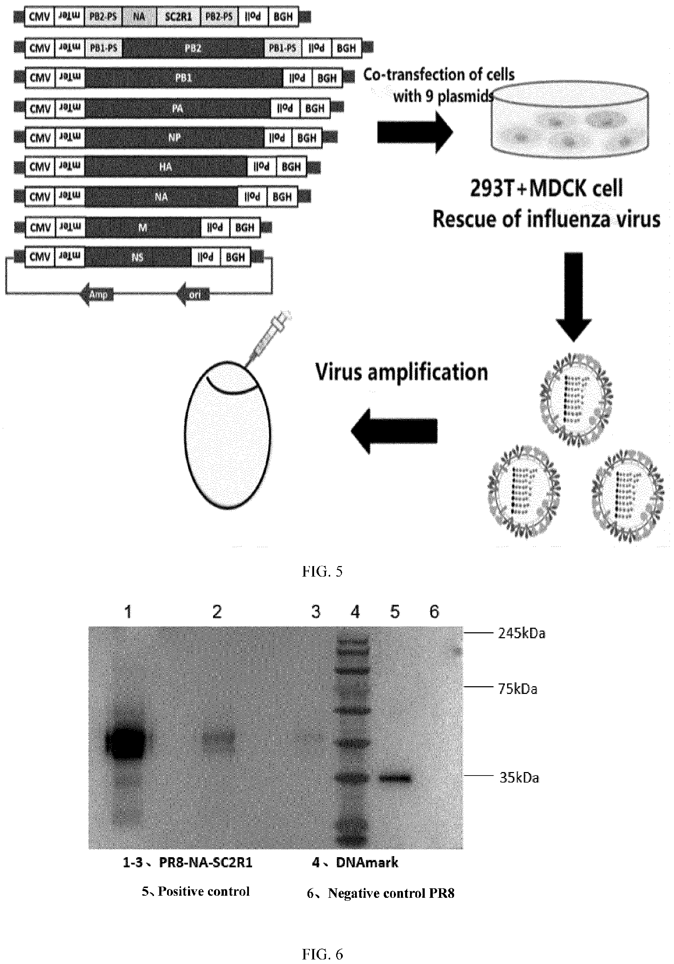

By means of an influenza virus reverse genetics system, the plasmids pMPR8-PB2-NA-SC2R1 and pMPR8-PB1-PB2 and the other seven influenza virus fragment plasmids (pMPR8-PB1, pMPR8-PA, pMPR8-NP, pMPR8-HA, pMPR8-NA, pMPR8-M, and pMPR8-NS) were co-transfected into mixed grown 293T and MDCK cells, and after 60 h, a mixed liquid of cells and supernatant was harvested. This mixed liquid was inoculated into a 9-day-old SPF chicken embryo, and the chicken embryo was placed in a biochemical incubator at 37°C for incubation; and after 72 h, the hemagglutination titer (HA) was determined, and a chicken embryo allantoic fluid was harvested, i.e. the rescued virus PR8-NA-SC2R1 (FIG. 5).

### 8 Verification of SC2R1 protein expression of the virus PR8-NA-SC2R1

The virus PR8-NA-SC2R1 was subjected to sucrose gradient centrifugation and purification, followed by WB detection. SC2R1 rabbit polyclonal antibody (SARS-CoV-2 (2019-nCoV) Spike RBD Antibody, Rabbit PAb, Antigen Affinity Purified, purchased from Sino Biological Inc.) was used as a primary antibody to detect the expression of SC2R1 protein. The purified virus was taken and added to PBS for 5-fold, 50-fold and 500-fold dilutions respectively, 5× loading buffer (containing β-mercaptoethanol) was added, and the mixtures were placed in a boiling water bath for 10 min to obtain linearized protein products. A protein gel (5% stacking gel + 10% separating gel) was prepared, the prepared sample was loaded into the protein gel, and after the processes of running on a gel, membrane transfer, blocking, membrane washing, incubation with primary antibody, incubation with secondary antibody, development, etc., the verification results of the SC2R1 protein expression of the virus PR8-NA-SC2R1 were obtained.

The verification results were as shown in FIG. 6, and the results showed that for the virus PR8-NA-SC2R1, SC2R1 protein expression could be detected.

### 9 Verification of HA protein expression of the virus PR8-NA-SC2R1

The virus PR8-NA-SC2R1 was subjected to sucrose gradient centrifugation and purification, followed by WB detection. HA rabbit polyclonal antibody (Influenza A H1N1 (A/Puerto Rico/8/1934) Hemagglutinin/HA Antibody, Rabbit PAb, Antigen Affinity Purified, purchased from Sino Biological Inc.) was used as a primary antibody to detect the expression of HA protein. The purified virus was taken for infecting an MDCK cell product, 5× loading buffer (containing β-mercaptoethanol) was added, and the mixture was placed in a boiling water bath for 10 min to obtain a linearized protein product. A protein gel (5% stacking gel + 10% separating gel) was prepared, the prepared sample was loaded into the protein gel, and after the processes of running on a gel, membrane transfer, blocking, membrane washing, incubation with primary antibody, incubation with secondary antibody, development, etc., the verification results of the HA protein expression of the virus PR8-NA-SC2R1 were obtained.

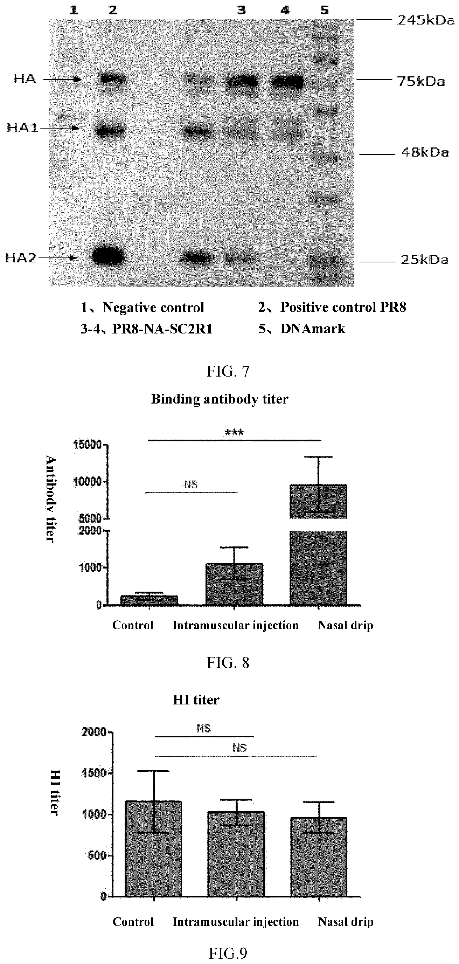

The verification results were as shown in FIG. 7, and the results showed that for the virus PR8-NA-SC2R1, HA protein expression could be detected.

### 10 Study on mouse immunogenicity of the virus PR8-NA-SC2R1

Mice were immunized with the vaccine PR8-NA-SC2R1 in different manners (nasal drip and intramuscular injection), and after immunization, blood was taken to detect the SC2R1 binding antibody titer and the HI titer. The results were as shown in FIG. 8, wherein SC2R1 binding antibody could be detected in both the nasal drip and intramuscular injection immunization groups of PR8-NA-SC2R1; and as shown in FIG. 9, HA antibody could be detected in both the nasal drip and intramuscular injection immunization groups of PR8-NA-SC2R1.

## Claims

1. A novel coronavirus vaccine based on an influenza virus vector, wherein the vaccine comprises an influenza virus vector loaded with a gene sequence encoding an S protein receptor binding region of a novel coronavirus; preferably, the amino acid sequence of the S protein receptor binding region is as set forth in SEQ ID NO. 3.

2. The vaccine according to claim 1, wherein a protein expressed by the amino acid sequence is capable of, in the human body,
inducing an immune response; or
generating a biological reporter molecule; or
generating a trace molecule for detection; or
regulating gene function; or
functioning as a therapeutic molecule.

3. The vaccine according to claim 1, wherein the vaccine is a DNA plasmid or an RNA expression plasmid.

4. The vaccine according to claim 1, wherein the influenza virus vector comprises a truncated PB2, PB 1, PA, NP, HA, NA, M or NS gene sequence, and the truncated gene sequence retains coding region packaging signal sequences at the 3' and 5' ends.

5. The vaccine according to any one of claims 1-4, wherein the 3' end of the nucleotide sequence of the gene encoding the S protein receptor binding region of the novel coronavirus is connected to a truncated fragment of NA gene, and the truncated fragment of the NA gene contains a coding region packaging signal at the 3' end.

6. The vaccine according to claim 5, wherein the amino acid sequence encoded by the truncated fragment of the NA gene is as set forth in SEQ ID NO. 1; preferably, the nucleotide sequence of the truncated fragment of the NA gene is as set forth in SEQ ID NO. 2.

7. The vaccine according to any one of claims 1-6, wherein the truncated fragment of the NA gene is connected to the nucleotide sequence of the gene encoding the S protein receptor binding region of the novel coronavirus via a linker sequence.

8. The vaccine according to any one of claims 1-7, wherein the influenza virus vector is derived from an influenza A or B strain, preferably an H1N1 subtype influenza strain.

9. The vaccine according to any one of claims 1-8, wherein the vaccine further comprises a pharmaceutically acceptable adjuvant, carrier, diluent or excipient.

10. A method for preparing a novel coronavirus vaccine based on an influenza virus vector, comprising the following steps:
(1) truncating at least one of PB2, PB1, PA, NP, HA, NA, M or NS genes of an influenza virus genome, retaining coding region packaging signal sequences at the 3' and 5' ends; and
(2) connecting the truncated fragment of the NA gene to the nucleotide sequence of a gene encoding an S protein receptor binding region of a novel coronavirus, then inserting the fragment obtained after connection into the truncated PB2, PB1, PA, NP, HA, NA, M or NS gene sequence of an influenza virus to obtain the novel coronavirus vaccine based on the influenza virus vector,
wherein preferably, the truncated fragment of the NA gene comprises a coding region packaging signal at the 3' end;
preferably, the amino acid sequence encoded by the truncated fragment of the NA gene is as set forth in SEQ ID NO. 1; and
preferably, the nucleotide sequence of the truncated fragment of the NA gene is as set forth in SEQ ID NO. 2.

11. The method according to claim 10, wherein the amino acid sequence of the S protein receptor binding region of the novel coronavirus is as set forth in SEQ ID NO. 3.
